# EUROPEAN PATENT APPLICATION

(11) **EP 2 708 202 A1**
(43) Date of publication of application: **19.03.2014**
(21) Application number: 13183956.5
(22) Date of filing: 11.09.2013
(51) Int. Cl.: A61B 19/00

(54) **Low profile, multi probe, cranial fixation device and method of use**

(30) Priority: 12.09.2012 US 201213612004
(71) Applicant: DePuy Synthes Products, LLC, Raynham, MA 02767-0350 (US)
(72) Inventor: Schorn, Gregory M., Raynham, Massachusetts 02767-0350 (US)
(74) Representative: Carpmaels & Ransford LLP

(57) **Abstract**

A system for securing an implantable probe or lead within a brain of a patient includes a burr hole cap, an elongated sheath, an elongated dilator and a plurality of elongated probes. The burr hole cap has an approximately cylindrical portion, a proximal end and a distal end. A flange portion of the burr hole cap is disposed at the proximal end. A septum is disposed within the cylindrical portion. The elongated sheath has a lumen extending therein. The elongated dilator is disposed within the lumen of the sheath. The dilator has a longitudinal length that is greater than the longitudinal length of the sheath. When the dilator is disposed in the lumen of the peelable sheath, the dilator and sheath may pierce into and through the septum. Each of the plurality of elongated probes are disposed within the lumen of the sheath. The probe has a longitudinal length that is greater than the longitudinal length of the sheath.

## Description

### BACKGROUND OF THE INVENTION

### 1. Field of the Invention

The invention relates to a device and methods for a low profile, multi probe, cranial fixation device.

### 2. Description of the Related Art

Neurocritical care monitoring in the hospital environment often requires the placement of multiple sensing probes and catheters in the brain to properly diagnose a patient's condition that has undergone traumatic brain injury or subarachnoid hemorrhage. Examples of probes that are used in neuro monitoring are, but not limited to, intracranial pressure (ICP), Brain Oxygen (Pb0₂), Cerebral Blood Flow (CBF) and Depth Electrode for EEG. Each probe or catheter is inserted through the burr hole in the skull, such that the probe or catheter is directed to reach its desired target site in the cerebral ventricle or parenchyma. The probes and catheters need to be secured in place in order to prevent their migration and dislodgement. Therefore, a device is required that can be secured to the patient's skull over the burr hole site and secure each probe and catheter in place.

It is therefore desirable to have a simpler and more accurate device and method for a device and methods for a low profile, multi probe, cranial fixation device.

### SUMMARY OF THE INVENTION

An object of the present invention is to provide a system for securing an implantable probe or lead within a brain of a patient includes a burr hole cap, an elongated sheath, an elongated dilator and a plurality of elongated probes. The burr hole cap has an approximately cylindrical portion, a proximal end and a distal end. A flange portion of the burr hole cap is disposed at the proximal end. A septum is disposed within the cylindrical portion. The elongated sheath has a lumen extending therein. The elongated dilator is disposed within the lumen of the sheath. The dilator has a longitudinal length that is greater than the longitudinal length of the sheath. When the dilator is disposed in the lumen of the peelable sheath, the dilator and sheath may pierce into and through the septum. Each of the plurality of elongated probes are disposed within the lumen of the sheath. The probe has a longitudinal length that is greater than the longitudinal length of the sheath.

A further object of the present invention is to provide a method of securing an implantable probe or lead within a brain of a patient that comprises the steps of:
securing a burr hole cap to a skull portion of the patient, the burr hole cap having an approximately cylindrical portion and a flange portion disposed at one end of the cylindrical portion, a septum disposed within the cylindrical portion;
inserting a dilator and a peelable sheath into and through the septum such that the dilator distal end pierces the dura;
removing the dilator from the peelable sheeth while leaving the peelable sheath in the septum;
inserting a probe into the peelable sheath until a distal end of the probe is located at a predetermined location within the brain;
removing the peelable sheath from the septum by moving the peelable sheath proximally; and
peeling away the peelable sheath from the probe.

### BRIEF DESCRIPTION OF THE DRAWINGS

In what follows, preferred embodiments of the invention are explained in more detail with reference to the drawings, in which:
FIG. 1 is a perspective exploded view showing the burr hole cap, sheath and a dilator according to one embodiment of the present invention;
FIG. 2 is a perspective view of the burr hole cap;
FIG. 3 is a perspective view of the sheath;
FIG. 3A is a cross-sectional view taken along lines 3A-3A of Fig. 3 and looking in the direction of the arrows;
FIG. 4A is a cross-sectional view showing the burr hole cap in place in a burr hole in the skull with the sheath and a dilator piercing through a septum in the burr hole cap;
FIG. 4B is a cross-sectional view showing the dilator being removed from the sheath;
FIG. 4C is a cross-sectional view showing a probe being passed through the sheath and placed in the desired position within the brain;
FIG. 4D is a cross-sectional view showing the dilator being removed from the probe and peeled away;
FIG. 4E is a cross-sectional view showing multiple probes piercing through the septum and having their distal end placed in the desired position within the brain;
FIG. 5 is a perspective view of the burr hole cap that has clips on its upper surface;
FIG. 6 is a perspective view of a sheath that has a single slit;
FIG. 6A is a cross-sectional view taken along line 6A-6A of Figure 6 and looking in the direction of the arrows;
FIG. 7 a perspective view of the sheath of Figure 6 with a probe placed therein; and
FIG. 8 is a front view of the sheath of Figure 6 showing the sheath being removed from the probe.

### DETAILED DESCRIPTION OF THE PRESENTLY PREFERRED EMBODIMENTS

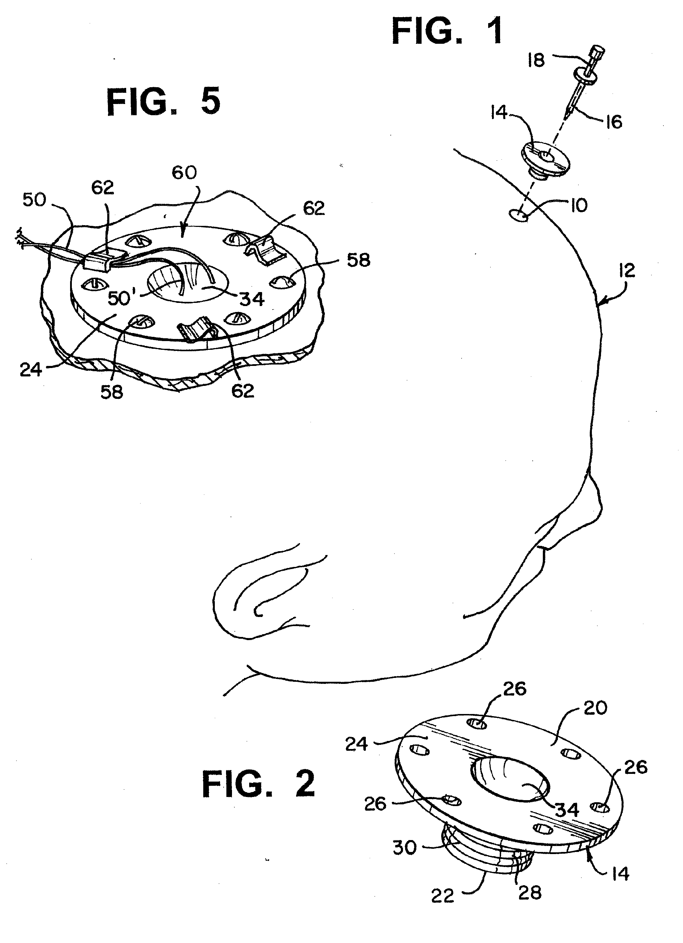

Referring now to Figure 1, a perspective exploded view showing a burr hole 10 in a patient 12. A burr hole cap 14, a sheath 16 and a dilator 18 are also illustrated.

Referring now to Figure 2, burr hole cap 14 is shown. Burr hole cap 14 has a proximal end 20 and a distal end 22. The proximal end 20 has a flange portion 24 that has a plurality of through holes 26 extending therethrough. The distal end 22 has a cylindrical projection 28 that has an outer surface, which is illustrated as having a threaded shape 30. The outer surface may have a smooth surface or a ridged surface. Extending through the central portion of burr hole cap 14 is a self sealing septum portion 34. A self sealing septum allows access to the brain using virtually any pointed tip tool while preventing body fluids from exiting the brain around the outside of the tool. Burr hole cap is preferably made of a low durometer (e.g., Shore 50A) silicone so that septum portion 34 of burr hole cap 14 instantly closes around any pointed tip tool that pierces the septum thereby providing a seal about the tool while inserted in the septum and a seal for the burr hole between the brain and outside the skull 60 after the tool is removed from the septum.

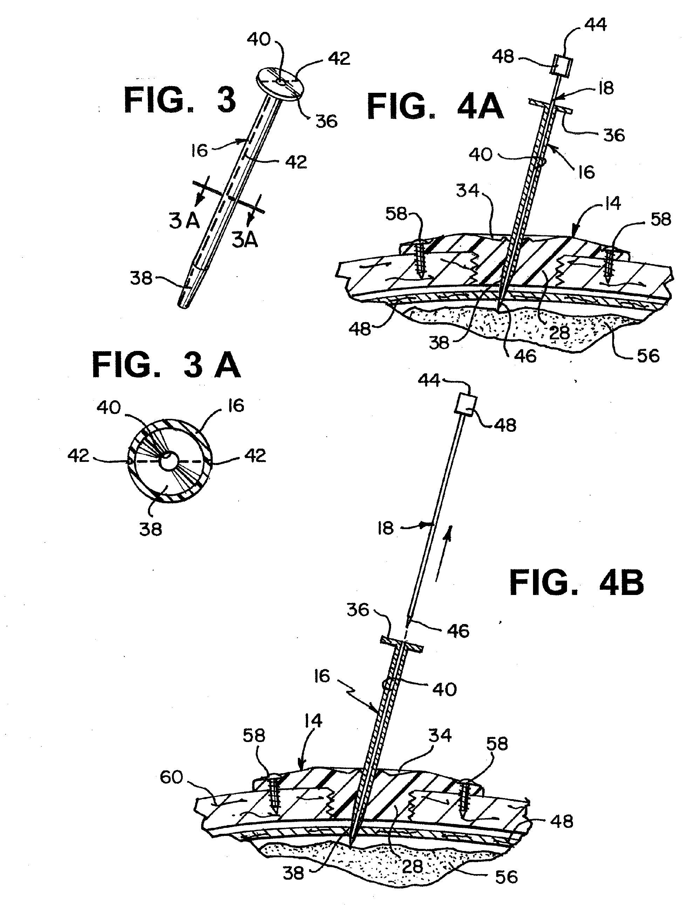

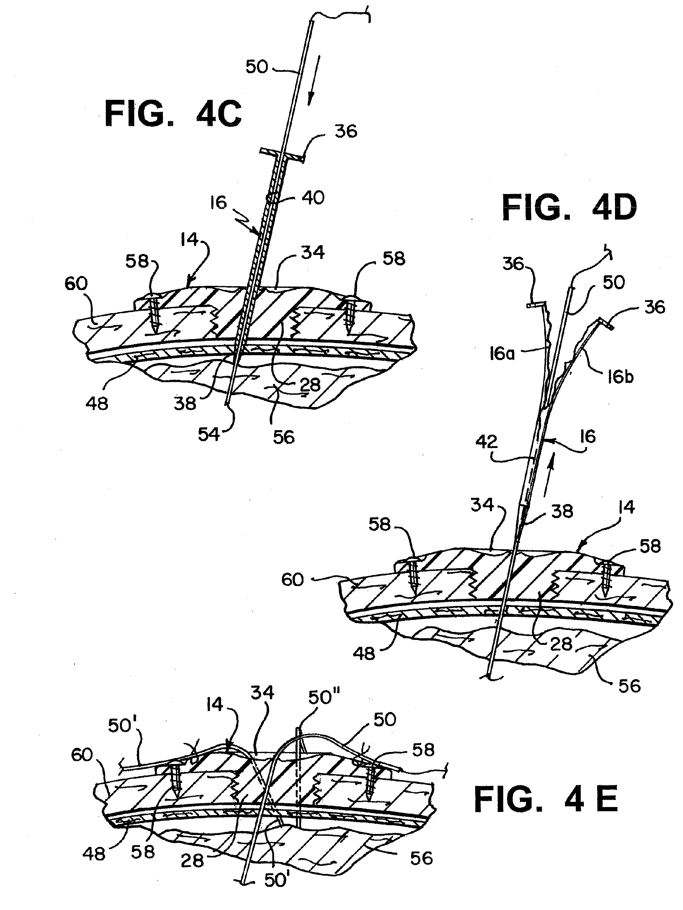

Referring now to Figure 3, a sheath 16 is illustrated. Sheath 16 has a proximal end 36 and a distal end 38. Sheath 16 has a lumen 40 extending through sheath 16 from the proximal end 36 to the distal end 38. Sheath 16 also has a pair of frangible connections 42 that are preferably diametrically opposed from each other to permit a user to peel sheath 16 into two pieces, as illustrated in Figure 4D. A dilator 18 is illustrated in Figures 1, 4A and 4B. Dilator has a proximal end 44 and a distal end 46. Proximal end 44 has an enlarged head 48 to facilitate grasping dilator 18 by the user. Head 48 is preferably cylindrical in shape, but may take other shapes in cross-section, such as, for example, square, hexagonal, etc. Distal end 46 of dilator 18 tapers to a point sufficient to pierce septum 34. As illustrated in Figures 1 and 4A, dilator 18 is inserted in the lumen 40 of sheath 16 so that the distal end 46 of dilator 18 extends beyond the distal end 38 of sheath 16.

To secure an implantable probe or lead within a brain of a patient the following steps may be followed:
A burr hole cap 14 is secured within a burr hole 10 to a skull portion of the patient 12;
A dilator 18 and a peelable sheath 16 are inserted into and through the septum 34 of the burr hole cap 14 such that the stylet distal end pierces the dura 48, as shown in Figure 4B. The dilator and sheath can be inserted at any given position and trajectory within the burr hole cap to reach a desired target site within the brain;
The dilator 18 is removed from the peelable sheeth 16 while leaving the peelable sheath 16 in the septum 18, as shown in Figure 4B;
A probe 50 is inserted into the peelable sheath 16 until a distal end 52 of the probe is located at a predetermined location 54 within the brain 56, as shown in Figure 4C;
The peelable sheath 16 is removed from the septum 34, and also the brain, by moving the peelable sheath proximally, while leaving the probe 50 in the septum 34 such that the distal end 52 of the probe is located at a predetermined location 54 within the brain, as shown in Figure 4D; and
The peelable sheath 16 is peeled into two pieces, 16a and 16b, away from probe 50 and are disposed of. Probe 50 remains in the septum 34 such that the distal end 52 of the probe is located at a predetermined location 54 within the brain 56, as shown in Figure 4D.

The user may now insert another assembled sheath and dilator into the septum should the user desire to place additional probes 50', 50", etc. at other predetermined locations within the brain. The steps above may be followed again to place additional probes within the brain as illustrated in Figure 4E.

To secure the burr hole cap 14 within burr hole 10, the user may use bone screws 58 that are placed through holes 26 in the flange portion 24 of burr hole cap 14 and into the skull 60, as illustrated in Figures 4A-4E.

Referring now to Figure 5, another embodiment of a burr hole cap 60 is illustrated. Burr hole cap 60 is similar to burr hole cap 14 except that cap 60 includes clips 62 disposed on its upper surface of flange portion 24. Probes 50, 50' may be guided into a desired position by being place under a clip 62 as desired by the user.

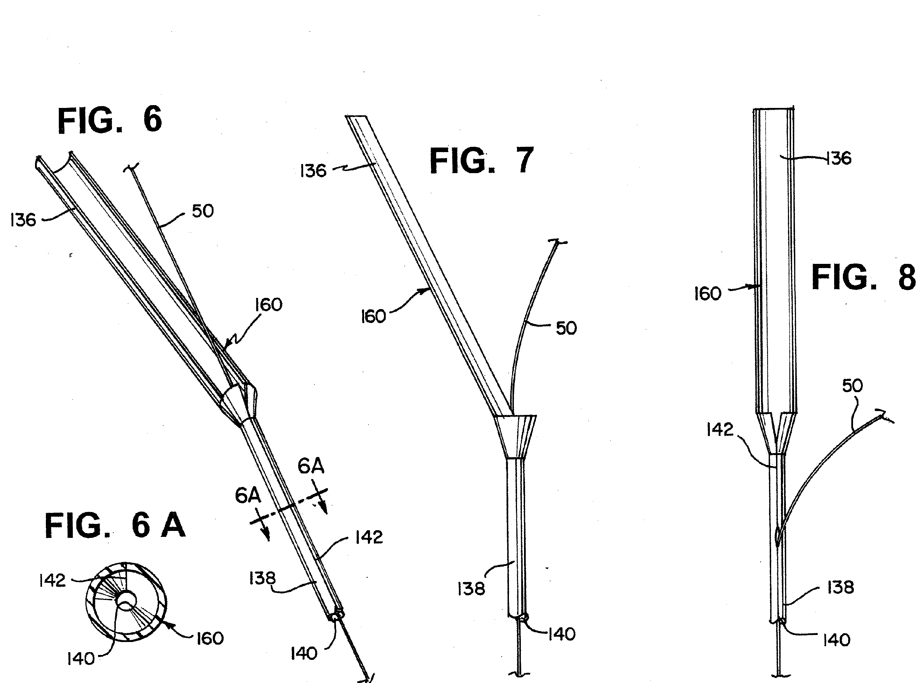

Referring now to Figures 6, 6A, 7 and 8, another embodiment of a sheath 160 is illustrated. Sheath 160 has a proximal end 136 and a distal end 138. Sheath 160 has a lumen 140 extending through sheath 160 from the proximal end 136 to the distal end 138. Sheath 160, at its distal end 138 is a split tube. In other words, sheath 160 has a radial cut 142 through the sheath. Cut 142 permits a user to peel sheath 160 away from probe 50, as illustrated in Figure 8, in a similar manner as described above with respect to Figures 4A-4D, but sheath 160 remains in one piece. Sheath 160 works with dilator 18 in a similar manner as sheath 16 as described above.

Thus, while there have been shown, described, and pointed out fundamental novel features of the invention as applied to preferred embodiments thereof, it will be understood that various omissions, substitutions, and changes in the form and details of the devices illustrated, and in their operation, may be made by those skilled in the art without departing from the spirit and scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

Every issued patent, pending patent application, publication, journal article, book or any other reference cited herein is each incorporated by reference in their entirety.

## Claims

1. A system for securing an implantable probe or lead within a brain of a patient comprising:
a burr hole cap having an approximately cylindrical portion, a proximal end and a distal end, a flange portion of the burr hole cap being disposed at the proximal end, a septum disposed within the cylindrical portion;
an elongated sheath having a lumen extending therein;
an elongated dilator capable of being disposed within the lumen of the sheath, the dilator having a longitudinal length that is greater than the longitudinal length of the sheath, when the dilator is disposed in the lumen of the peelable sheath the dilator and sheath are capable of piercing into and through the septum; and
a plurality of elongated probes, each of the plurality of probes being capable of being disposed within the lumen of the sheath, the probe having a longitudinal length that is greater than the longitudinal length of the sheath.

2. The system according to claim 1 wherein the sheath has a first portion and a second portion that are connected by a frangible connection.

3. The system according to claim 1 wherein the sheath has a radial cut.

4. The system according to claim 1 wherein the flange portion of the burr hole cap has a proximal surface, at least one tab being disposed on the proximal surface.

5. The system according to claim 1 wherein the flange portion of the burr hole cap has at least one through hole.

6. A method of securing an implantable probe or lead within a brain of a patient comprising the steps of:
securing a burr hole cap to a skull portion of the patient, the burr hole cap having an approximately cylindrical portion and a flange portion disposed at one end of the cylindrical portion, a septum disposed within the cylindrical portion;
inserting a dilator and a peelable sheath into and through the septum such that the dilator distal end pierces the dura;
removing the dilator from the peelable sheeth while leaving the peelable sheath in the septum;
inserting a probe into the peelable sheath until a distal end of the probe is located at a predetermined location within the brain;
removing the peelable sheath from the septum by moving the peelable sheath proximally; and
peeling away the peelable sheath from the probe.

7. The method of claim 6, further comprising the steps of:
inserting a second dilator and a second peelable sheath into and through the septum such that the dilator distal end pierces the dura;
removing the second dilator from the second peelable sheeth while leaving the second peelable sheath in the septum;
inserting a second probe into the second peelable sheath until a distal end of the probe is located at a second predetermined location within the brain, the second predetermined location being spaced from the first predetermined location;
removing the second peelable sheath from the septum by moving the second peelable sheath proximally; and
peeling away the second peelable sheath from the probe.

8. The method of claim 7, further comprising the steps of:
inserting a third dilator and a third peelable sheath into and through the septum such that the dilator distal end pierces the dura;
removing the third dilator from the second peelable sheeth while leaving the third peelable sheath in the septum;
inserting a third probe into the third peelable sheath until a distal end of the probe is located at a third predetermined location within the brain, the third predetermined location being spaced from the first and the second predetermined locations;
removing the third peelable sheath from the septum by moving the third peelable sheath proximally; and
peeling away the third peelable sheath from the probe.

9. The method of claim 6, wherein the step of removing the peelable sheath includes separating the peelable sheath into two portions.

10. The method of claim 6, further comprising the steps of:
placing a portion of the probe under a clip on a surface of the flange portion of the burr hole cap.
